# EUROPEAN PATENT APPLICATION

(11) **EP 2 181 997 A1**
(43) Date of publication of application: **05.05.2010**
(21) Application number: 08382060.5
(22) Date of filing: 30.10.2008
(51) Int. Cl.: C07D 471/14, C07D 471/04

(54) **A process for the preparation of tadalafil**

(71) Applicant: Chemo Ibérica, S.A., 08028 Barcelona (ES)
(72) Inventor: Marras, Giovanni, 28100, Novara (Novara) (IT); Rasparini, Marcello, 27010, Cura Carpignano (PV) (IT); Castaldi, Graziano, 28072, Briona (NO) (IT)
(74) Representative: ZBM Patents

(57) **Abstract**

The present invention relates to a process for the preparation of Tadalafil, to intermediates for the synthesis thereof, and to a process for their preparation.

## Description

### Field of the invention

The present invention relates to a process for the preparation of Tadalafil and intermediates for the synthesis thereof.

### Background of the invention

Tadalafil is a potent and selective phosphodiesterase type 5 (PDE5) enzyme inhibitor. The inhibition of PDE5 blocks the degradative action of PDE5 on cyclic guanosine monophosphate (cGMP). The inhibition of PDE5 enhances erectile function by increasing the amount of cGMP. cGMP relaxes smooth muscle and increases blood flow to the corpus cavernosum. Tadalafil is an orally administered drug therefore currently used in the treatment of male erectile dysfunction and is marketed world-wide under the trade name of Cialis^{®}. Tadalafil is the third impotence pill after Sildenafil (Viagra^{®}) and Vardenafil (Levitra^{®}). Although they all are PDE5 inhibitors, Tadalafil's distinguishing pharmacologic feature is its longer half-life (17.5 hours) compared with Viagra^{®} and Levitra^{®} (4-5 hours). This longer half-life results in a longer duration of action and is, in part, responsible for the Cialis^{®} nickname of the "weekend pill". This longer half-life is also the basis of current investigation for Tadalafil's use in pulmonary arterial hypertension as a once-daily therapy. Tadalafil is a compound of formula (I) 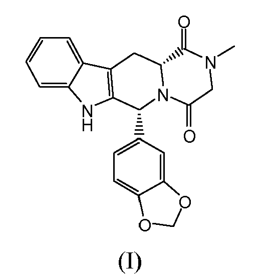 chemically known as (6*R*-trans)-6-(1,3-benzodioxol-5-yl)-2,3,6,7,12,12a-hexahydro-2-methyl-pyrazino [1',2':1,6] pyrido [3,4-*b*] indole-1,4-dione, which is disclosed in US 5 859 006.

Several methods have been used to prepare Tadalafil.

The process disclosed in the patent US 5 859 006 (Scheme 1) involves condensation of D-tryptophan methyl ester with a piperonal derivative to yield a compound of formula (II). After conversion into a thioamide derivative of formula (III), cyclization occurs in presence of both an alkylating and reducing agents to provide a tetrahydro-β-carboline derivative of formula (IV), which on treatment with chloroacetyl chloride and methyl amine, gives Tadalafil. The compound of formula (IV) can also be obtained in one step, after separation of the other diastereoisomer, by a Pictet Spengler reaction between D-tryptophan methyl ester and piperonal in presence of an acid, such as trifluoroacetic acid.

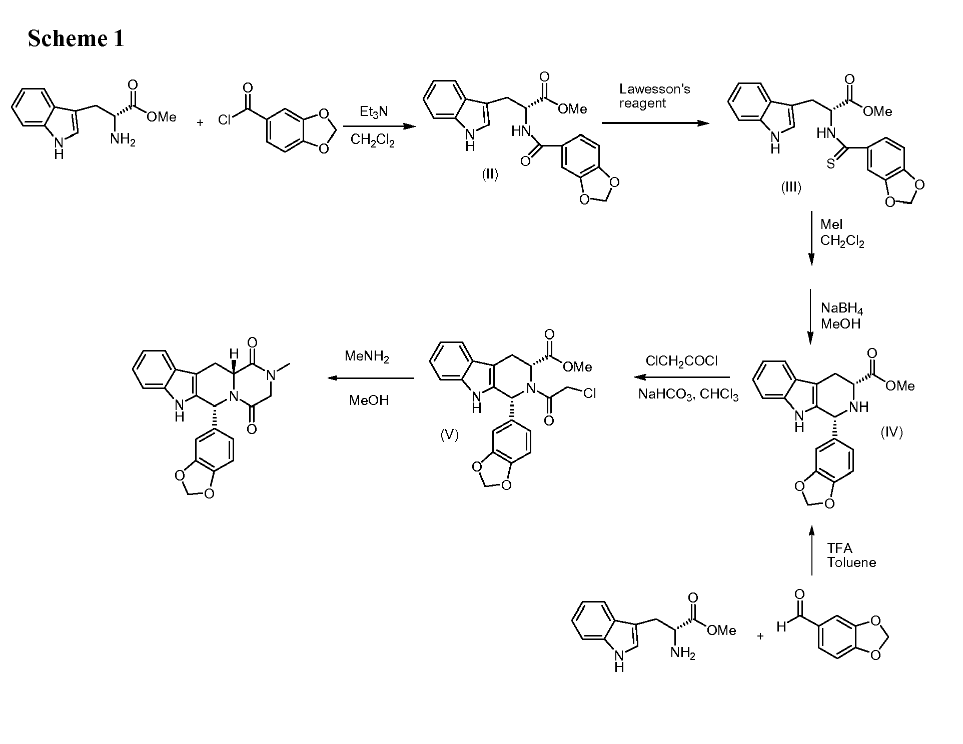

The patent application WO2007/10038 discloses the reaction of D-tryptophan with piperonal to provide a tetrahydro-β-carboline acid that was cyclised to Tadalafil in presence of a sarcosine derivative.
The patent application WO2007/1107 discloses the reaction of D-tryptophan methyl amide with piperonal, to provide an intermediate that after reaction with chloroacetyl chloride cyclises to Tadalafil in presence of butyllithium.
Thus, the active substance prepared by the processes known up till now can only be obtained in a satisfactory quality after running through a large number of process steps. Moreover a toxic alkylating agent, such as methylamine, is often used.
There is the need of an alternative synthesis for industrial application, which involves a fewer number of stages and makes use of safer reagents.
An object of the present invention is to provide an efficient and commercially useful process for the manufacture of Tadalafil, that avoids the above-identified problems.

### Summary of the invention

It has now been found a process for the preparation of Tadalafil in a straightforward manner exploiting novel intermediates.

The method of the present invention involves a process for preparing Tadalafil (I) or a pharmaceutically acceptable salt, hydrate, solvate or polymorph thereof, 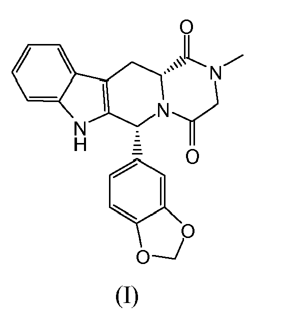 which comprises:
hydrogenating a compound of formula (VII) with the (*R*)-configuration at both stereocenters, 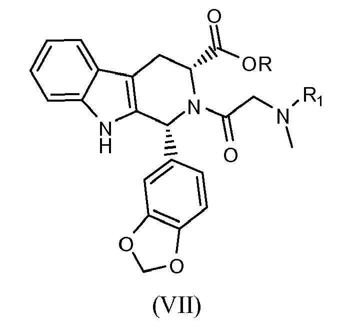
wherein:
R is a C₁-C₆ alkyl group or an aryl group, optionally substituted;
R₁ is a group that can be removed by hydrogenolysis; in the presence of a catalyst.

In another embodiment, the invention provides the synthesis of the compound of formula (VII) by reaction of a compound of formula (VI) with the (*R*)-configuration at both stereocenters, 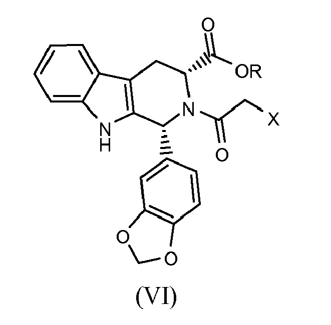 wherein:
R is a C₁-C₆ alkyl group or an aryl group, optionally substituted;
X is a leaving group;
   with a secondary amine R₁MeNH, wherein R₁ is a group that can be removed by hydrogenolysis, in presence of a base.

In another embodiment, the invention provides a compound of formula (VII), as defined above, as well as its hydrates, solvates, polymorphs and pharmaceutically acceptable salt.

In another embodiment, the invention provides the use of compounds of formula (VII) as intermediates in the preparation of Tadalafil.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The term "C₁-C₆ alkyl" refers to a straight or branched hydrocarbon having from 1 to 6 carbon atoms. Examples of alkyl groups include, without limitation, methyl, ethyl, *n-*propyl, isopropyl, *n*-butyl, *sec*-butyl, isobutyl, tert-butyl, *n-*pentyl, *n*-hexyl, and the like. The C₁-C₆ alkyl group may be optionally substituted. A preferable C₁-C₆ alkyl group of the present invention is methyl.

The term "aryl" refers to an aromatic carbocyclic group having a single ring (e.g., phenyl), multiple rings (e.g. biphenyl), or multiple condensed rings in which at least one is aromatic (e.g. 1,2,3,4-tetrahydronaphtyl, 1-naphtyl, 2-naphtyl, anthryl, phenanthryl). The aryl group may be optionally substituted. A preferable aryl group of the present invention is benzyl.

The term "halogen" refers to chlorine, bromine, fluorine or iodine.

The term "substituted" refers to groups substituted with from 1 to 5 substituents selected from the group consisting of C₁-C₆-alkyl, halogen, aryl, and the like.

The term "leaving group" refers to one of the groups chloride, bromide, iodide, mesylate, tosylate, triflate; preferably chloride or bromide, more and more preferably chloride.

The term "about" encompasses the range of experimental error that may typically occur in a measurement.

The term "diastereomerically pure" refers to a sample containing greater than about 95% of the desired diastereoisomer, preferably greater than about 98% of the desired diastereoisomer, and more preferably greater than about 99.5% of the desired diastereoisomer.

The term "pharmaceutically acceptable salt(s)", as used herein, unless otherwise indicated, includes salts of acidic or basic groups which may be present in the compounds of the present invention. The compounds prepared according to the present invention that are basic in nature are capable of forming a wide variety of salts with various inorganic and organic acids. The acids that may be used to prepare pharmaceutically acceptable acid addition salts of such basic compounds of are those that form non-toxic acid addition salts i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate and pamoate [i.e., 1,1'-methylene-bis-(2-hydroxy-3-naphthoate)] salts. The compounds prepared according to the present invention that include a basic moiety, such as an amino group, may form pharmaceutically acceptable salts with various amino acids, in addition to the acids mentioned above.

The term "solvate" refers to a molecular complex comprising a disclosed or claimed compound and a stoichiometric or non-stoichiometric amount of one or more solvent molecules (e.g., ethanol).

The term "hydrate" refers to a solvate comprising a disclosed or claimed compound and a stoichiometric or non-stoichiometric amount of water.

The term "polymorph" refers to substances that have the same chemical formula, but different crystal structures.

Generally, the chemical transformations described throughout the specification may be carried out using substantially stoichiometric amounts of reactants, though certain reactions may benefit from using an excess of one or more of the reactants. Additionally, many of the reactions disclosed throughout the specification, may be carried out at room temperature, but particular reactions may require the use of higher or lower temperatures, depending on reaction kinetics, yields, and the like. Furthermore, many of the chemical transformations may employ one or more compatible solvents, which may influence the reaction rate and yield. Depending on the nature of the reactants, the one or more solvents may be polar protic solvents, polar aprotic solvents, non-polar solvents, or some combination.
The compounds obtained by the chemical transformations of the present invention, can be used for the following steps without further purification or can be effectively separated and purified by employing a conventional method well known to those skilled in the art, such as recrystallization, column chromatography, or by transforming then into a salt or by washing with an organic solvent or with an aqueous solution, eventually adjusting pH.

In accordance with Scheme 2, the present invention provides a process for the preparation of Tadalafil (I). The method of the present invention makes use of generally inexpensive and readily available reagents. 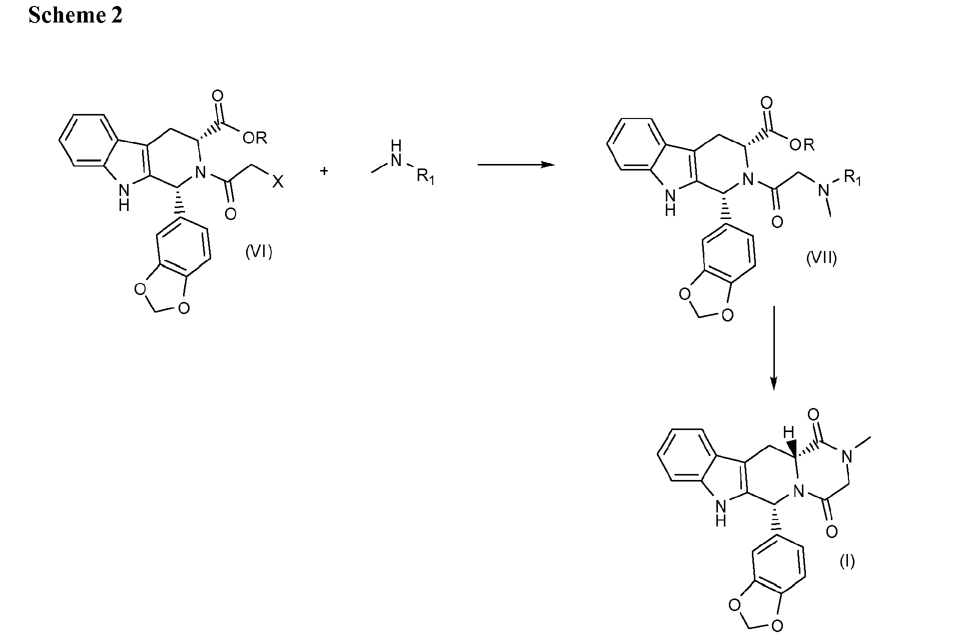 wherein:
R is a C₁-C₆ alkyl group or an aryl group, optionally substituted;
X is a leaving group;
R₁ is a group that can be removed by hydrogenolysis.

The process of the present invention comprises the catalytic hydrogenation of a compound of Formula (VII) to produce Tadalafil (I) (Scheme 2). A diastereomerically pure compound of Formula (VII) may be obtained by reaction of the tetrahydro-β-carboline of formula (VI) with a secondary amine R₁MeNH in presence of a base. The two steps of the present invention occur without epimerization at both stereocenters.

In one embodiment, the invention provides the hydrogenation of the compound of formula (VII), or a pharmaceutically acceptable salt, hydrate, solvate, or polymorph thereof, to obtain Tadalafil (I), in the presence of a catalyst. This reaction allows the deprotection and ring closure steps to occur simultaneously and thus reduces the number of steps.
The hydrogenation can be carried out by reaction with hydrogen gas, applied at pressures ranging from atmospheric pressure to 50 atm. Preferably, the hydrogen pressure is from 1 to 3 atm.
Any suitable hydrogenation catalyst known to those skilled in the art can be used for hydrogenation. Suitable hydrogenation catalyst is a transition metal, for instance Pd or Ni, preferably Raney^{®} Ni, Ni pellets. The amount of metal to be employed can vary within wide limits. Preferably, the concentration of the metal is between 2% molar and 10% molar with respect to substrate.
Any suitable solvent known to those skilled in the art can be used for hydrogenation of a compound of Formula (VII). Suitable solvents are polar aprotic solvents, such as dimethylacetamide, dimethylformamide, N-methylpyrrolidinone, ethyl acetate; and polar protic solvents, such as alcohols. Examples of alcohols include, but are not limited to, methanol, ethanol, isopropanol, *tert*-butanol; preferably ethanol.
The hydrogenation is done at a suitable temperature. A suitable temperature is a temperature in order for the reaction to be complete. The temperature may range from room temperature to the reflux temperature; preferably the temperature is between 60 and 100°C.

Compounds of formula (VII) are novel compounds and as such, are also object of the present invention. Compounds of formula (VII) are compounds wherein:
R is a C₁-C₆ alkyl group or an aryl group, optionally substituted; and R₁ is a group that can be removed by hydrogenolysis. Any suitable group that can be removed by hydrogenolysis known to those skilled in the art can be used. A suitable group that can be removed by hydrogenolysis is a benzyl group, optionally substituted. Preferably, R₁ is benzyl.

A preferred example of compound (VII) is a compound wherein R is methyl and R₁ is benzyl: (1*R*,3*R*)-methyl 1-(benzo[*d*][1,3]dioxol-5-yl)-2-(2-(benzyl(methyl)amino)acetyl)-2,3,4,9-tetrahydro-1*H*-pyrido[3,4-*b*]indole-3-carboxylate.

In another embodiment, the invention provides a process for preparing a diastereomerically pure compound of formula (VII), or a pharmaceutically acceptable salt, hydrate, solvate, or polymorph thereof, by reacting a tetrahydro-β-carboline of formula (VI) with the (R)-configuration at both stereocenters, wherein R is a C₁-C₆ alkyl group or an aryl group, optionally substituted, preferably R is methyl; X is a leaving group, preferably chloride or bromide, more preferably chloride; with a secondary amine R₁MeNH, wherein R₁ is as above defined, in presence of a basic agent, in a suitable solvent, such as a polar aprotic solvent, for example N,N-dimethylacetamide, dimethylformamide, ethyl acetate.

A basic agent is an organic base, for example straight or branched tertiary amines, such as triethylamine, diisopropyl ethyl amine, tetramethylethylenediamine, tributylamine, pyridine, N-methylmorpholine, tetramethylurea, methylpyrrolidinone, 4-dimethylaminopyridine, proton sponge or dimethylaniline; or an inorganic base, for example alkali or alkali earth hydroxides, such as sodium hydroxide, calcium hydroxide, or alkali or alkaline-earth metal carbonates, such as sodium or potassium carbonate. Preferably the base is an inorganic base, more preferably potassium carbonate. The preferred base is employed in a molar ratio comprised between 1.0 and 2.0 to the compound (VI), preferably between 1.0 and 1.5, more preferably between 1.0 and 1.2. The secondary amine R₁MeNH is employed in a molar ratio comprised between 1.0 and 2.0 to the compound (VI), preferably in a molar ratio between 1.0 and 1.2.

The secondary amines R₁MeNH are commercially available compounds or may be prepared according to methods well known in the art. The compounds useful according to the invention may be prepared, unless specifically specified, by the application or adaptation of known methods, by which are meant methods used heretofore or described in the literature, patents or patent applications, the Chemical Abstracts and on the Internet. The known compound of formula (VI) may be prepared as described in the patent application WO95/19978.

While the present invention has been described in terms of its specific embodiments, certain modifications and equivalents will be apparent to those skilled in the art and are included within the scope of the present invention.
In the following, the present invention shall be illustrated by means of some examples, which are not construed to be viewed as limiting the scope of the invention.

### Examples

The following abbreviations refer respectively to the definitions below:
DMA (N,N-dimethylacetamide); K₂CO₃ (potassium carbonate).

### Example 1

**(1*R*,3*R*)-methyl-1,2,3,4-tetrahydro-2-(2-(benzyl(methyl)amino)acetyl)-1-(3,4-methylenedioxyphenyl)-9*H*-pyrido[3,4-*b*]indole-3-carboxylate (VII)**

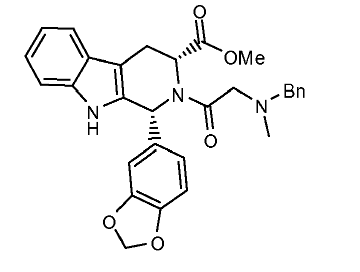

A 50 mL three-necked flask fitted with thermometer and reflux condenser was charged with (1*R*,3*R*)-methyl 1,2,3,4-tetrahydro-2-chloroacetyl-1-(3,4-methylenedioxyphenyl)-9*H-*pyrido [3,4-*b*] indole-3-carboxylate (VI) (1.39 g, 3.26 mmol), DMA (5.33 mL), K₂CO₃ (0.5 g, 3.6 mmol) and N-benzylmethylamine (0.41 mL, 3.26 mmol). The resultant solution was stirred at room temperature. After 2 hours, the mixture was poured in brine (20 mL) and extracted with isopropyl acetate. The combined organic phases were washed with brine (3 x 5 mL), dried over sodium sulfate and concentrated to a residue under reduced pressure, affording 1.5 g of the desired product (VII), as a white solid. Yield: 70%.
¹H NMR (d₆-DMSO 300 MHz, 298K) 2.24 (s, 3H), 2.94-3.00 (m, 5H), 3.44-3.68 (m, 3H), 5.56 (bd, *J* = 6.4, 1H), 5.95 (s, 1H), 5.96 (s, 1H), 6.55 (bd, *J* = 7.4, 1H), 6.75 (bs, 1H), 6.77 (d, *J* = 8.0, 1H), 6.84 (bs, 1H), 7.05 (td, *J* = 7.4, 0.9, 1H), 7.12 (td, *J* = 7.5, 1.2, 1H), 7.17-7.32 (m, 6H), 7.56 (d, *J* = 7.7, 1H), 10.76 (bs, 1H)
¹³C NMR (d₆-DMSO 75.4 MHz, 298K) 21.9, 42.5, 51.3, 51.9, 52.4, 61.0, 61.7, 101.5, 107.0, 108.0, 109.8, 111.8, 118.5, 119.2, 122.0, 123.0, 126.7, 127.7, 128.7, 129.6, 131.1, 134.7, 137.1, 138.6, 147.1, 147.5, 170.6, 171.5

### Example 2

**(6*R*-trans)-6-(1,3-benzodioxol-5-yl)-2,3,6,7,12,12a-hexahydro-2-methyl-pyrazino [1',2':1,6] pyrido [3,4-*b*] indole-1,4-dione (Tadalafil) (I)** 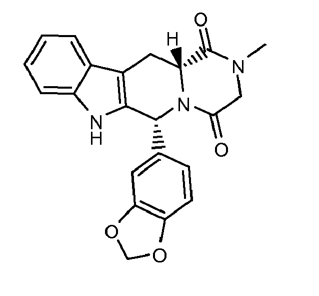 Under H₂ atmosphere (3 atm) and magnetic stirring, Raney^{®} Ni (2800 slurry in water, 0.0276 g, 0.47 mmol), previously washed with methanol (3 times), was added to a solution of (1*R*,3*R*)-methyl-1,2,3,4-tetrahydro-2-(2-(benzyl(methyl)amino)acetyl)-1-(3,4-methylenedioxyphenyl)-9*H*-pyrido[3,4-*b*]indole-3-carboxylate (VII) (3.00 g, 4.70 mmol) in DMA (21.3 mL). The mixture was heated at 90°C for 17 hours and then cooled to room temperature. The suspension was filtered over a pad of Celite^{®} and the resulting solutionand the resulting solution was concentrated until 6 mL. Methanol (12 mL) was added and the solid which was so obtained was filtered over Buchner, washed with methanol (4 mL) and oven-dried under reduced pressure for 2 hours, affording 1.3 g of the title compound, as a white solid. Yield: 70%
¹H NMR (d₆-DMSO 300 MHz, 298K): 2.91-3.00 (m, 4H), 3.32 (s, 1H), 3.47-3.54 (dd, *J* = 4.6, 11.3, 1H), 3.93 (d, *J* = 17.1, 1H), 4.17 (d, *J* = 17.1, 1H), 4.35-4.40 (dd, *J* = 4.27, 11.6, 1H), 5.91 (s, 2H), 6.11 (s, 1H), 6.76 (s, 2H), 6.85 (s, 1H), 6.98-7.06 (m, 2H), 7.28 (d, *J* = 7.9, 1H), 7.52 (d, *J* = 7.3, 1H), 11.0 (s, 1H)
¹³C NMR (d₆-DMSO 75.4 MHz, 298K) 23.8, 33.4, 52.0, 55.9, 56.1, 101.5, 105.3, 107.6, 108.6, 111.9, 118.7, 119.5, 119.9, 121.8, 126.4, 134.5, 136.8, 137.6, 146.7, 147.6, 167.1, 167.5

## Claims

1. A process for preparing Tadalafil of formula (I), or a pharmaceutically acceptable salt, hydrate, solvate or polymorph thereof, 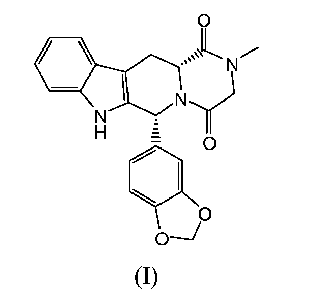 which comprises:
hydrogenating a compound of formula (VII) with the (*R*)-configuration at both stereocenters, 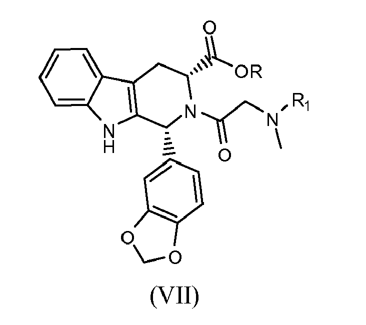
wherein:
R is a C₁-C₆ alkyl group or an aryl group, optionally substituted with from 1 to 5 substituents selected from the group consisting of C₁-C₆-alkyl, halogen, and aryl;
R₁ is a group that can be removed by hydrogenolysis; in the presence of a catalyst.

2. The process according to claim 1, wherein R is methyl and R₁ is benzyl.

3. The process according to claim 1, wherein the catalyst is a transition metal.

4. The process according to claim 3, wherein the transition metal is Pd or Ni, preferably Ni, more preferably Raney^{®} Ni, Ni pellets.

5. The process according to claim 1, wherein the hydrogenation is conducted under hydrogen pressure from atmospheric pressure to 50 atm, preferably the hydrogen pressure is from 1 to 3 atm.

6. The process according to claim 1, wherein the hydrogenation is conducted in a polar aprotic solvent, preferably N,N-dimethylacetamide, dimethylformamide; or in a polar protic solvent, such as alcohols, preferably ethanol.

7. The process according to claim 1, wherein the hydrogenation is conducted a temperature from room temperature to the reflux temperature; preferably the temperature is between 60°C to 100°C.

8. A process for preparing a compound of formula (VII) by reaction of a compound of formula (VI) with the (*R*)-configuration at both stereocenters, 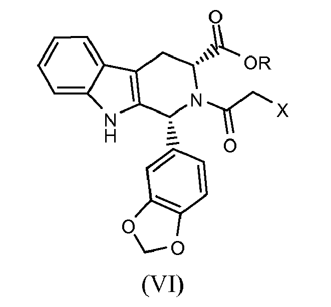 wherein:
R is a C₁-C₆ alkyl group or an aryl group, optionally substituted with from 1 to 5 substituents selected from the group consisting of C₁-C₆-alkyl, halogen, and aryl;
X is a leaving group;
with a secondary amine R₁MeNH, wherein R₁ is a group that can be removed by hydrogenolysis, in presence of a base.

9. The process according to claim 8, wherein:
R is methyl;
X is chloride or bromide, preferably chloride;
R₁ is benzyl.

10. The process according to claim 8, wherein the base is an organic base or an inorganic base, preferably an inorganic base, more preferably potassium carbonate.

11. The process according to claim 8, wherein the reaction is conducted in a polar aprotic solvent, such as N,N-dimethylacetamide, dimethylformamide, ethyl acetate.

12. A compound of formula (VII) with the (*R*)-configuration at both stereocenters, 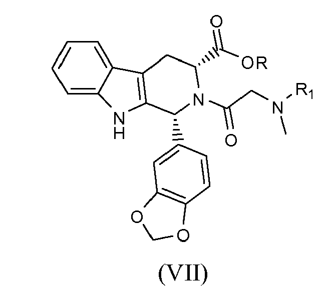 wherein:
R is a C₁-C₆ alkyl group or an aryl group, optionally substituted with from 1 to 5 substituents selected from the group consisting of C₁-C₆-alkyl, halogen, and aryl;
R₁ is a group that can be removed by hydrogenolysis;
as well as its hydrates, solvates, polymorphs and pharmaceutically acceptable salt.

13. The compound according to claim 12, wherein R is methyl and R₁ is benzyl.

14. Use of compounds of formula (VII) as defined in any one of claims 12-13, as intermediates in the preparation of Tadalafil.
